# EUROPEAN PATENT APPLICATION

(11) **EP 4 679 450 A1**
(43) Date of publication of application: **14.01.2026**
(21) Application number: 24186998.1
(22) Date of filing: 08.07.2024
(51) Int. Cl.: G16H 40/67, G16H 50/20, A61B 5/00, H04L 67/12

(54) **DATA SOURCE SELECTION**

(71) Applicant: Koninklijke Philips N.V., 5656 AG Eindhoven (NL)
(72) Inventor: HUIJBREGTS, Laurentia Johanna, Eindhoven (NL); VAN LIESHOUT, Ron Martinus Laurentius, Eindhoven (NL); NEUMANN, Rolf, 5656AG Eindhoven (NL); BULUT, Murtaza, Eindhoven (NL)
(74) Representative: Philips Intellectual Property & Standards

(57) **Abstract**

The disclosed method and system select a data source from multiple data sources, each configured to generate data for determining a value of the same monitoring parameter of a subject. The selection process involves determining a fitness score for each data source and selecting an updated primary data source based on these scores. The selection is biased towards maintaining the current primary data source. Accordingly, the invention provides a selection process that is designed to minimize unnecessary switching between data sources, thereby reducing potential confusion for healthcare professionals and enhancing the reliability of the monitoring parameter values.

## Description

### FIELD OF INVENTION

The present invention relates to the field of medical monitoring technology, and specifically to methods and systems for selecting a data source among multiple data sources for determining a value of a same monitoring parameter of a subject.

### BACKGROUND

Monitoring systems are a cornerstone of modern healthcare, providing real-time data related to one or more subjects, such as physiological parameters of subjects. These systems typically rely on a multitude of data sources, each designed to provide values from which monitoring parameters such as heart rate, blood pressure, or respiratory rate can be derived. As technology advances, the number of sensors available, and the number of parameters that can be monitored, is continually increasing. This leads to a wealth of data that can be used to inform patient care.

Whilst each data source may provide values which can be used to determine different parameters, many data sources can also be used to derive values of the same parameter. Of course, the values from different data sources generally do not result in the exact same monitoring parameter value, for example due to intrinsic sensor differences, differences in the base medical data generated from which the monitoring parameter may be derived, and differences in the algorithms that derive the parameter value. This can lead to different levels of reliability and accuracy among the data sources.

In a monitoring system, values of a monitoring parameter are used in various ways. They can be displayed on a screen, used to trigger alarms when they fall within an alarm range, used to output sounds at a frequency or pitch that correlates with the measured parameter, or used as input for algorithms that generate further monitoring parameters, scores etc. For these uses, it is often desired or even mandatory to have a single input value per parameter per timeframe. This necessitates a choice of which data source to use, or a combination of inputs from various data sources into a single value.

In any case, healthcare professionals rely on these values to guide their decisions. Therefore, it is of utmost priority that the values they see reflect real changes. Accordingly, it is important to appropriately select the data source for the monitoring parameter.

### SUMMARY OF INVENTION

The invention is defined by the claims.

According to an aspect of the present disclosure, a method for selecting a data source amongst a plurality of data sources is provided. Each data source is configured to generate data suitable for determining a value of a same monitoring parameter of a subject. The plurality of data sources comprises a primary data source that is a currently selected source of the monitoring parameter value and at least one secondary data source that is not a currently selected source of the monitoring parameter value. The method includes determining a fitness score of each of the plurality of data sources and selecting an updated primary data source amongst the plurality of data sources based on the fitness scores. The selection of the updated primary data source is biased toward maintaining the primary data source as the current source of the monitoring parameter.

Proposed concepts thus aim to provide schemes, solutions, concepts, designs, methods, and systems pertaining to the selection of a data source among multiple data sources each capable of generating data suitable for determining a value of the same monitoring parameter of a subject. In particular, embodiments aim to provide a selection process designed to minimize unnecessary switching between data sources, thereby reducing potential confusion for healthcare professionals and enhancing the reliability of the monitoring parameter values.

The disclosed method involves determining a fitness score for each data source, which may reflect the suitability of the data source for generation of the monitoring parameter. The fitness score of a data source may therefore reflect one or more of a reliability and accuracy of the data it generates, subject/clinician preferences (e.g., comfort, operability, etc.), data source footprint (e.g., power consumption, required bandwidth, etc.). The selection of the primary data source, i.e., the currently selected source of the monitoring parameter value, is then updated based on these fitness scores. Importantly, the selection process is biased towards maintaining the current primary data source, thereby minimizing unnecessary switching between data sources.

By biasing the selection process toward maintaining the primary data source as the currently selected source avoids unnecessary switching. For example, it may cause unnecessary confusion and require unnecessary effort to switch to a secondary data source that has a marginally greater fitness score than the primary data source, with the benefit of such a switch being negligible. Thus, proposed embodiments provide that the switching only occurs when there is a clear benefit associated with switching, it is convenient to switch, and/or when it is unlikely to cause confusion.

In other words, by asserting that the selection of the updated primary data source is biased toward maintaining the primary data source as the current source of the monitoring parameter, it is imposed that there must be a clear benefit and/or it must be convenient to switch the data source. Thus, switching to a data source that provides a negligible advantage over the current data source, or may only briefly provide an advantage, is avoided. Accordingly, when a primary data source and a secondary data source are found to be equally suitable (or the secondary data source is found to be marginally more suitable) in a given scenario, use of the primary data source is maintained. Nevertheless, if it is found that the secondary data source is substantially more suitable in a given scenario, and/or there is a benefit and switching is both convenient and unlikely to cause confusion, then switching is performed.

The disclosed system and method offer several advantages. By minimizing unnecessary switching between data sources, they reduce potential confusion for healthcare professionals and enhance the reliability of the monitoring parameter values. Furthermore, by considering a range of factors in determining the fitness score of each data source, they ensure that the selected data source is the most suitable in the given context. This approach may ensure continuity in the generation of the monitoring parameter value, reducing the likelihood of misinterpretation due to unnecessary, inappropriate, and abrupt changes in the source of the monitoring parameter.

In another aspect of the present disclosure, the method may include assessing a switching criterion describing a condition during which switching of the currently selected source of the monitoring parameter value is facilitated, responsive to the fitness score of one of the secondary data sources exceeding the fitness score of the primary data source. If the switching criterion is met, the secondary data source is selected as the updated primary data source. That is, switching between the primary data source and the secondary data source is biased toward maintaining the status quo unless the switching criterion is met simultaneous with the secondary data source having a greater fitness score than the primary data source. Indeed, by imposing a switching criterion, switching from the primary data source to the secondary data source may only occur when it is particularly advantageous and/or convenient. This approach allows for a dynamic selection of the data source, not only taking into account the relative fitness scores of the data sources, but also whether certain criteria are met.

In yet another aspect of the present disclosure, the switching criterion may comprise a fitness variance threshold, a value variance threshold, and/or a fitness failsafe threshold. The fitness variance threshold imposes that the score of the secondary data source exceeds the fitness score of the primary data source by a predetermined difference. The value variance threshold imposes that a difference between the value of the monitoring parameter determined based on data from the secondary source and the value of the monitoring parameter determined based on data from the primary source is less than a predetermined difference. The fitness failsafe threshold imposes that the fitness score of the primary data source fails to meet a predetermined condition.

These thresholds provide a quantitative basis for determining when to switch from the primary data source to a secondary data source, thereby enhancing the reliability and consistency of the monitoring parameter value.

In some embodiments, the switching criterion may be based on a medical context of the subject, a status and/or condition of the subject, an alarm context, and/or a workflow context. Accordingly, the switching criterion is adapted based on the present conditions and need, ensuring more appropriate switching from the primary data source to the secondary data source.

In a further aspect of the present disclosure, the fitness score of each data source may be based on a confidence score indicating an accuracy measure of data generated by the respective data source and/or an accuracy measure of the value of the monitoring parameter determined based on data from the respective data source. This approach ensures that the data source with the greatest accuracy measure (e.g., accuracy, sensitivity, specificity, precision, etc.) is prioritized, thereby enhancing the reliability of the monitoring parameter value.

In additional embodiments of the invention, the method may further comprise determining the confidence score based on analyzing data from the data source, and/or based on analyzing data source context information. In particular, the data source context information may comprise expected source quality information, historical source quality information, sensor source placement information, sensor source use information, source interference information, and/or perceived source quality information. Thus, the accuracy of the value from the data sources may be reliably assessed.

Additionally or alternatively, the fitness score of each data source may be based on at least one of patient comfort information, patient status/condition information, patient clinical information, system footprint information, data delay information, and future clinical suitability information associated with the respective data source. This approach ensures that the data source with that is most appropriate/suitable given various factors (such as delay, system footprint, etc.) is prioritized, thereby enhancing the suitability of the monitoring parameter value to the given context.

Furthermore, selecting the updated primary data source may be further based on primary data source historical use information. That is, the data source currently selected as the source of the monitoring parameter value may also consider previous use of data sources. For example, it may be beneficial to consider the length of time that the primary data source has been used as the source of the monitoring parameter value. This may help to avoid rapidly repeated switching of the data source.

In another aspect of the present disclosure, the method may include delaying switching the currently selected source of the monitoring parameter values to the updated primary data source based on contextual use information associated with the monitoring parameter values. This approach ensures that the timing of the switch does not interfere with the interpretation of the monitoring parameter values. In particular, there may be particular circumstances during which switching would be disadvantageous, such as mid procedure, or when a healthcare professional is midway through interpretation of the data.

In yet another aspect of the present disclosure, the method may include at least one of displaying the values from the primary data source; generating an interface output based on the values from the primary data source; generating an alarm based on values from the primary data source; providing the values from the primary data source of the monitoring value to a clinical algorithm; and/or modifying an operating state of secondary data sources.

According to a further aspect of the present disclosure, a computer program is provided. The computer program comprises computer program code means adapted, when said computer program is run on a computer, to implement a method for selecting a data source amongst a plurality of data sources as described herein.

According to another aspect of the present disclosure, a system for selecting a data source amongst a plurality of data sources is provided. Each data source is configured to generate data suitable for determining a value of a same monitoring parameter of a subject. The plurality of data sources comprises a primary data source that is a currently selected source of the monitoring parameter value and at least one secondary data source that is not a currently selected source of the monitoring parameter value. The system includes an assessment unit configured to determine a fitness score of each of the plurality of data sources, and a source selection unit configured to select an updated primary data source amongst the plurality of data sources based on the fitness scores. Selecting the updated primary data source is biased toward maintaining the primary data source as the current source of the monitoring parameter.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a method for selecting a data source amongst multiple data sources, according to aspects of the present disclosure;
FIG. 2 depicts a flow diagram of an exemplary embodiment of the method for selecting a data source;
FIG. 3 depicts a flow diagram of another exemplary embodiment of the method for selecting a data source;
FIG. 4 presents a system for selecting a data source amongst multiple data sources interfacing with a monitoring system, according to aspects of the present disclosure; and
FIG. 5 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

The disclosed method and system select a data source from multiple data sources, each configured to generate data for determining a value of the same monitoring parameter of a subject. The selection process involves determining a fitness score for each data source and selecting an updated primary data source based on these scores. The selection is biased towards maintaining the current primary data source. Accordingly, the invention provides a selection process that is designed to minimize unnecessary switching between data sources, thereby reducing potential confusion for healthcare professionals and enhancing the reliability of the monitoring parameter values.

The selection of the updated primary data source is biased towards maintaining the primary data source as the current source of the monitoring parameter, thereby minimizing the potential for confusion or misinterpretation of data by healthcare professionals.

By way of explanation, a health care professional should be guided only by real changes of the subject and not by changes that arise when switching from one data source to another, which may be misinterpreted as real changes. For example, the heart rate of the subject may be derived from two different data sources, such as from an optical sensor and an electrical sensor. However, there may be differences in heart rate determined based on values from these sensors. Therefore, when switching between these different sources of the heart rate, it may appear that the subject's heart rate has changed, whilst in reality their heart rate has remained the same. Therefore, switching from one data source to another should be minimized.

Switching between data sources that provide data from which the same monitoring parameter of the subject may be derived may cause a change in the value of the monitoring parameter due to intrinsic sensor differences and/or differences in algorithms that derive the parameter value from the data. Such a change may be misinterpreted by the clinician as a physiological change. This should be prevented, because clinicians often base their decisions on changes in shown parameter values and/or changes in waveforms.

In existing systems, data from multiple data sources are received, and the data source which is expected to give the most reliable value may be prioritized for selection. The selection is thus independent of which data source had been used before. Some systems allow automatic fall backs for when the data source with highest priority is unavailable. The selection is independent of which data source had been used before, and the suitability of the potential data source is also not considered for switching.

Accordingly, it is proposed that a system (such as patient monitor) that receives/retrieves data from which a monitoring parameter may be derived from more than one source, prioritizes one sensor/algorithm over the other sensor/algorithm(s). Specifically, the currently selected data source is provided with an advantage to stay selected during a selection process, unless the new data source is particularly advantageous and/or it is convenient to switch and unlikely to cause confusion.

In the proposed selection process, there are at least two competing aspects. The first aspect is if the data source that has been the one in use up to now (as this is a positive indication to stay with the same sensor). That is, the data source that is currently selected is prioritized for future selection. In other words, selection is biased toward maintaining the present/current data source as the selected/used data source (for any use of the monitoring parameter, such as visualization, alarming, scoring etc.). The second aspect is the relative suitability of the data source to the present context. In other words, a fitness score of each data source, reflecting appropriateness of use of the data source.

Specifically, the fitness score of each data source may reflect various factors such as whether the monitoring parameter is directly or indirectly derivable from data from the data source, precision and accuracy of the data from the data source and the resultant monitoring parameter, subject comfort (i.e., how comfortable it is for the subject whilst retrieving data using the data source), data source footprint (e.g., power consumption, processing power, storage capacity, required communication bandwidth), battery status particularly when the data source is a wireless sensor), data delay, and clinical considerations (e.g., suitability of the data source in the near future, subject context, subject health status, clinician workflow). All these second aspects may differ between data sources (e.g., sensors, algorithms and combinations thereof).

Subject comfort may refer to how comfortable (e.g., invasive, painful, inconvenient etc.) it is to gather the data using the data source. For example, when measuring blood pressure, an arm cuff may be used which typically inflates on the arm. The inflation may cause discomfort, and therefore alternative data sources may be preferred (e.g., a finger cuff, or an arm cuff operated with a lesser degree of inflation or for a shorter time duration). Furthermore, in cases where one data source is a multiparameter sensor and the other data source is a single-parameter sensor, the multiparameter sensor may be preferred since the number of sensors and/or cables are reduced, thus improving subject comfort. The precision and accuracy aspect above is time-varying and may be directly determined by prior art techniques, such as by using signal to noise ratio. The accuracy may also be derived from indirect measurements of factors that are expected to influence the accuracy of a particular data source, (e.g. patient health status, patient posture, activity and motion of the body part comprising the sensor, parameter range, environmental conditions like room temperature, electromagnetic interference, ambient light levels and resulting impact to optical measurements), which might be based on explicit ambient sensor data or implicit based on context information. Throughout this disclosure, the accuracy may be referred to as a key part of the fitness score. Nevertheless, the invention holds if the fitness score is based on any of the above factors, or any combination thereof.

The values derived from data from the primary data source can be used for one or more of display on the screen, alarm generation, multisensory outputs such as sound and vibration, and use as input for an algorithm used for scoring subject health (e.g., hemodynamic stability index, or early warning score computation). Furthermore, any data source not identified as the primary data source may be put into a sleep, standby or off state if the data from the data source is not being used. Of course, other uses of the data from the primary data source are contemplated, and would be readily apparent to the skilled person.

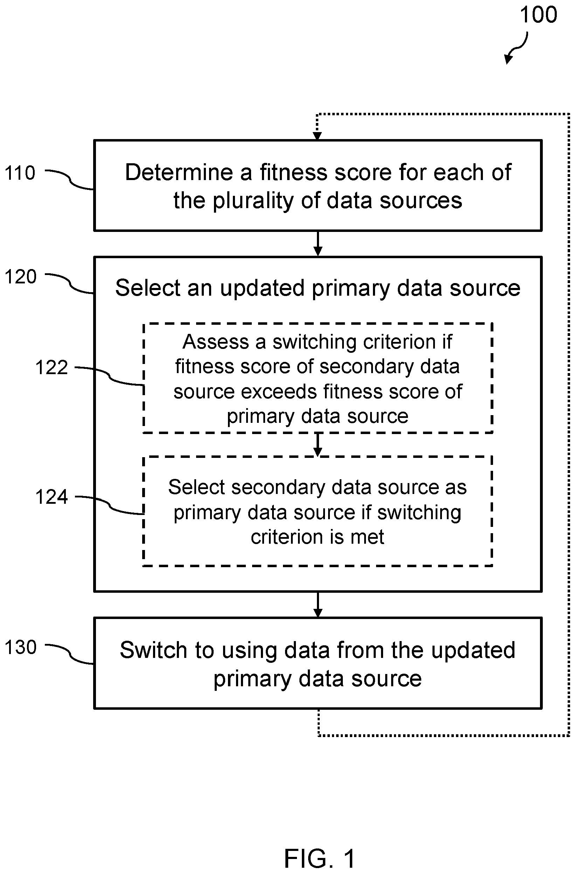

Referring to FIG. 1, there is presented a flow diagram of a method for selecting a data source amongst multiple data sources, according to aspects of the present disclosure. That is, there is provided a method for choosing which medical data source amongst a plurality of medical data sources to use for a certain purpose.

The purpose may be simply for displaying a value of a monitoring value derivable from data from the data source. Alternatively, the selected data source may be used for generation and storage of a monitoring parameter, such as a health score. Of course, many other uses of data from the data source are considered.

To be clear, each data source may be a sensor generating data in real time, an algorithm that receives data from a data source and provides processed data, a data storage which provides historical data previously generated by a data source, or a combination thereof. In essence, any means adapted to produce data that can be used to determine values of a monitoring parameter may be considered a data source.

In the case of the present invention, each data source is configured to generate data suitable for determining a value of a same monitoring parameter of a subject, such as heart rate, blood pressure, oxygen saturation, and so on. Accordingly, each data source from which the data source will be selected produces data that can be used to derive the monitoring parameter of the subject. By way of example, the monitoring parameter may be a heart rate, and the plurality of data sources may comprise a chest-strap heart rate monitor, an optical wrist mounted heart rate monitor, and one or more ECG gel electrodes sensors.

To be clear, in some cases each data source may be an algorithm suitable for generating a value of a same monitoring parameter of a subject. In some cases, a same algorithm may be considered to be multiple different data sources given that it is used in different ways (e.g., a different floating window, sample rate, etc.). As a result, each of the algorithms may have different associated advantages (e.g., different accuracies, etc.). For example, given the monitoring parameter is a respiration rate, the plurality of data sources may include different algorithms using ECG and PPG data. If the floating window of the algorithm is increased the accuracy will go up (e.g. less sensitive to motion artifacts), but with a loss of coverage (i.e. less frequent data points). There is thus a balance to be found, with the desired balance dependent on the clinical situation and/or user preferences (e.g., in the OR/ICU the coverage is essential as changes are expected to be frequent and fast, whilst monitoring parameters generated for require less coverage but in some cases higher accuracy).

The plurality of data sources comprise a primary data source that is a currently selected source of the monitoring parameter value. As such, the primary data source is the data source that is currently being used to determine/derive the monitoring parameter value for the purpose of the selection. The plurality of data sources also comprise at least one secondary data source that is not a currently selected source of the monitoring parameter value. Of course, any of the secondary data sources may currently be selected for use for another purpose.

The method 100 for selecting a data source amongst a plurality of data sources begins with step 110, in which a fitness score for each of the plurality of data sources is determined. The fitness score may be any metric by which the suitability of the data source for a given purpose in a given context may be assessed.

In some cases, the fitness score may be based on various factors such as the accuracy and/or precision of data generated by the respective data source, accuracy and/or precision of the monitoring parameter value derivable from the generated data, patient comfort information, system footprint information, data delay information, and future clinical suitability information associated with the respective data source.

In some aspects, the fitness score may be based on a confidence score indicating an accuracy of data generated by the respective data source. The confidence score may be determined by analyzing the data from the data source, taking into account factors such as the signal-to-noise ratio, the precision and accuracy of the measurements, and the reliability of the data source. This allows the method 100 to assess the quality of the data generated by each data source and assign a fitness score accordingly.

Specifically, the fitness score may also be based on data source context information. This information may include expected source quality information, historical source quality information, sensor source placement information, sensor source use information, source interference information, and/or perceived source quality information. For example, the expected source quality information may provide an indication of the expected performance of the data source based on known characteristics or specifications of the data source. The historical source quality information may provide an indication of the past performance of the data source, which can be useful in predicting future performance. The sensor source placement information may provide information about the placement of the sensor on the subject, which can affect the quality of the data generated by the sensor. The sensor source use information may provide information about how the sensor is being used, which can also affect the quality of the data. The source interference information may provide information about any interference that may be affecting the data generated by the data source. The perceived source quality information may provide an indication of the perceived quality of the data source based on subjective assessments or evaluations. More particularly, the perceived source quality information may relate to personal preference (i.e., an inclination to use one data source over another data source as default), and therefore this may depend on the user.

In additional and/or alternative cases, the fitness score may also be based on at least one of patient comfort information, system footprint information, data delay information, and future clinical suitability information associated with the respective data source. For example, a data source that provides a high level of patient comfort, has a small system footprint, provides data with low delay, and is suitable for future clinical scenarios may be assigned a higher fitness score. This allows the method 100 to consider a wide range of factors in selecting the primary data source, ensuring that the selected data source is not just accurate, but also suitable for the specific requirements of the subject and the healthcare setting.

The patient comfort information may provide an indication of the comfort level of the subject when using the data source, which can affect the quality of the data generated by the data source. The system footprint information may provide an indication of the impact of the data source on the system resources, such as power consumption, processing power, storage capacity, and communication bandwidth. The data delay information may provide an indication of the delay in the data generated by the data source (e.g., from frequency of measurement, duration of measurement, processing time, and transfer time of the data), and/or for a detectable change in the physiological parameter to be derivable from the data generated by the data source (e.g., for a change in concentration of a biomarker in the patient's blood, to show up in the biomarker concentration measured in sweat, takes quite some time) which can affect the timeliness and relevance of the data. The future clinical suitability information may provide an indication of the suitability of the data source for future clinical scenarios, which can affect the long-term usefulness of the data source.

In step 120, an updated primary data source amongst the plurality of data sources is selected based on the fitness scores. This step of selecting the updated primary data source is biased toward maintaining the primary data source as the current source of the monitoring parameter. Accordingly, if the fitness scores of the data sources indicate that there is likely only a small benefit/advantage to switch the primary data source (or they are substantially equivalent), the current primary data source will remain selected. This biasing towards maintaining the primary data source can help minimize potential confusion or misinterpretation of data by healthcare professionals that arises when switching between data sources.

In some cases, if the fitness score of a secondary data source exceeds that of the primary data source, a switching criterion is assessed 122. The switching criterion describes a condition during which switching of the currently selected source of the monitoring parameter value is facilitated. In other words, the switching criterion may include various thresholds and conditions that guide the decision to switch from the primary data source to the secondary data source. Accordingly, in only some cases may the primary data source be altered. This therefore biases the selection process toward maintaining the selected primary data source even when another data source has a higher fitness score, as the switching criterion imposes one or more restrictions for switching.

If the switching criterion is met, the secondary data source is selected 124 as the updated primary data source. This selection process allows for the possibility of switching to a more suitable data source when the conditions defined by the switching criterion are met, while still maintaining a bias towards the current primary data source.

The switching criterion may include a fitness variance threshold in some embodiments. The fitness variance threshold imposes that the score of the secondary data source exceeds the fitness score of the primary data source by a predetermined difference. This ensures that a switch to a secondary data source is made when there is a substantial improvement in the fitness score, thereby minimizing unnecessary switches between data sources.

In some aspects, the switching criterion may also or alternatively include a value variance threshold. This threshold imposes a condition that a difference between the value of the monitoring parameter determined based on data from the secondary source and the value of the monitoring parameter determined based on data from the primary source is less than a predetermined difference. This helps to ensure that the switch between data sources does not result in a drastic change in the value of the monitoring parameter, which could potentially confuse healthcare professionals.

By way of example, if the primary data source and the secondary data source produce values that are in different ranges (one indicates slightly elevated, another indicates normal, for example) or correspond to different alarm conditions, then switching the primary data source is not allowed. If they fall within the same range or alarm condition, then switching may be facilitated. This is because it is often the case that healthcare professionals pay more attention to the category of the values, rather than the absolute values of the monitoring parameter.

In some embodiments, the switching criterion may include a fitness failsafe threshold. This threshold imposes a condition that the fitness score of the primary data source fails to meet a predetermined condition. This can serve as a safeguard to ensure that the primary data source is switched when its fitness score falls below an acceptable level, regardless of the fitness score of the secondary data source.

Of course, other criteria for switching would be apparent to the skilled person, and would depend upon particular implementation of the invention.

Furthermore, the switching criterion may be based on various contextual factors. This may alter the threshold that needs to be met to allow switching of the data source. Indeed, in some circumstances switching may be relatively inconsequential (such as when no one is paying attention, or during setup of the monitoring system). Such factors that may be used to adjust the switching criterion may include a medical context of the subject, a status and/or condition of the subject, an alarm context, and/or a workflow context. For example, the switching criterion may take into account the current health status of the subject, the urgency of the alarm situation, or the workflow requirements of the healthcare professionals. This allows the method 100 to adapt to the specific circumstances of the subject and the healthcare setting, providing a more personalized and efficient monitoring solution.

In some aspects, the selection of the updated primary data source may be further based on primary data source historical use information. This information may provide an indication of the past use of the primary data source, which can be useful in predicting future performance and reliability. For example, the historical use information may include information about how long the primary data source has been in use, how often it has been used, and how recent its use has been. This information can help to assess the reliability and stability of the primary data source, and can be used to bias the selection towards maintaining the primary data source as the current source of the monitoring parameter.

In a specific implementation of the above, each of the data sources may be assigned a score (i.e., points) based on a selection of the above mentioned factors. For example, points may be awarded to the data source that is currently selected (e.g. if so, this adds 10 points). Then, points are added for each of the aspects related to suitability of the secondary data sources, and may be weighted based on suitability of switching in the present moment. For example, if the accuracy of a data source is very high, this adds 15 points, if the accuracy is medium it adds 8 points, and if the accuracy is very low, it adds no points. The data source that is selected is therefore the data source with the highest number of points/the highest score. This is another means by which the selection of the primary data source may be biased to maintain the primary data source as the currently selected data source, as initial points are awarded to the currently selected data source.

Finally, the method 100 involves switching 130 to using data from the updated primary data source. This switch may be implemented in various ways depending on the specific requirements of the system and the nature of the data sources. For example, the switch may involve physically changing the connection to a different sensor, or it may involve changing the software configuration to use data from a different algorithm. It may also involve altering an operating state of the secondary data sources, in order to save power, memory, etc.

The method 100 may involve delaying the switching 130 of the currently selected source of the monitoring parameter values to the updated primary data source based on contextual use information associated with the monitoring parameter values. This delay can be beneficial in situations where an immediate switch could potentially confuse or mislead healthcare professionals. For example, if the subject is undergoing a medical procedure or if the healthcare professionals are in the process of making a clinical decision based on the current monitoring parameter values, an immediate switch could potentially disrupt these activities. By delaying the switch, the method 100 can provide a smoother transition between data sources, thereby minimizing potential confusion or misinterpretation of data.

In some cases, the method 100 may involve switching the data source in the background to allow training or adaptation of algorithms making use of the data source. This can be particularly useful in situations where the algorithms require a period of adaptation or training before they can generate accurate and reliable results. By switching the data source in the background, the method 100 can ensure that the algorithms have sufficient time to adapt or train on the new data, thereby improving the accuracy and reliability of the monitoring parameter values generated by the algorithms.

In some aspects, the method 100 may involve at least one of displaying the values from the primary data source, generating an interface output based on the values from the primary data source, generating an alarm based on values from the primary data source, providing the values from the primary data source of the monitoring value to a clinical algorithm, and/or modifying an operating state of secondary data sources. These various uses of the values from the primary data source can provide a comprehensive monitoring solution that can be tailored to the specific requirements of the subject and the healthcare setting.

Of course, the method 100 may be performed continuously in order to automatically and constantly ensure that the primary data source is the most appropriate data source given the present context.

In some aspects, the method 100 may be implemented in a computer system or a medical monitoring device, and may be used to select the data source for various types of monitoring parameters. The method 100 may also be used in various healthcare settings such as hospitals, clinics, home care, and so on.

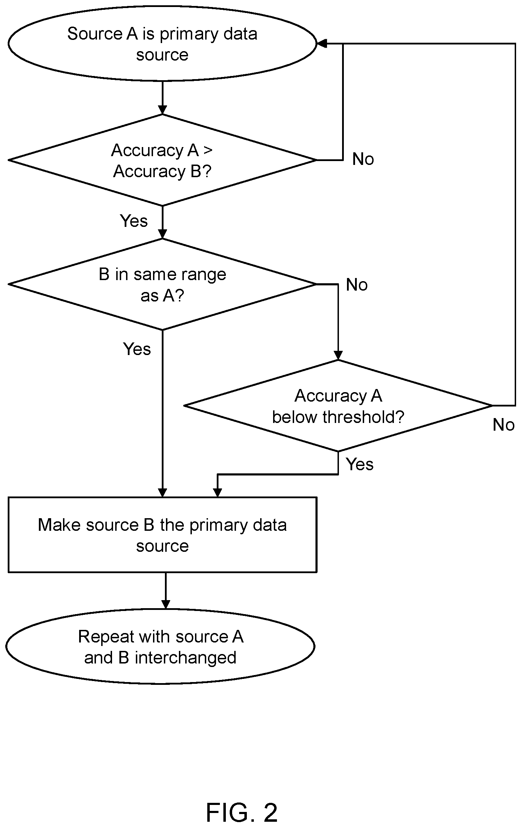

Referring to FIG. 2, a specific method for selecting a primary data source among multiple data sources is depicted. In this example, the fitness score of each data source is entirely based on the confidence score of the data source describing the accuracy of the data source. It will be appreciated that additional or alternative factors dictating the suitability of the data source may be used.

In some aspects, the method begins with source A designated as the primary data source. The process involves comparing the accuracy of source A to source B. The accuracy of each data source may be determined based on various factors such as the quality of the data generated by the respective data source, the reliability of the data source, and other factors that may affect the accuracy of the data.

In some cases, if the accuracy of source A is greater than that of source B, the method checks if source B is in the same range as source A. The range may refer to a specific range of values for the monitoring parameter, a classification of the monitoring parameter, or any other categorization of the monitoring parameter values. The determination of whether source B is in the same range as source A may be based on various factors such as the values of the monitoring parameter generated by the respective data sources, the accuracy of these values, and other factors that may affect the categorization of the monitoring parameter values.

To be clear, the range referred to herein may refer to a variety of different ranges, within which values of the monitoring parameter typically lead to the same predictive algorithm output. The range therefore may refer to one or more of
(i) an alarm range. For example, the monitoring the parameter may be in a no-alarm range, a yellow alarm range, or a red alarm range, each indicating a level of alarm related to the monitoring parameter;
(ii) a goal range. The monitoring parameter may be within or outside the goal (i.e., target) range;
(iii) an early warning score (EWS / NEWS / MEWS). The early warning score may be an integer output related to the monitoring parameter, where each different integer relates to a certain action that should be followed;
(iv) a deterioration prediction. The monitoring parameter value is input to an algorithm that will either predict a stabile situation, or a deterioration (such as hemodynamic instability with HSI);
(v) a cause classification. The monitoring parameter value is input to an algorithm that may determine the likely cause for a condition or deterioration; and
(vi) a visualization range. On a monitoring device, the way in which the monitoring parameter value is presented may depend on whether it is below a lower threshold, between a lower threshold and a higher threshold, or above a higher threshold.

If source B is in the same range as source A, in some aspects, source B is made the primary data source, and the process repeats with source A and B interchanged. This allows for a smooth transition between data sources, minimizing potential confusion or misinterpretation of data by healthcare professionals.

If source B is not in the same range as source A, the method checks if the accuracy of source A is below a threshold in some cases. The threshold may be a predetermined value or range of values that represents an acceptable level of accuracy for the data source. If the accuracy of source A is below the threshold, source B is made the primary data source. This ensures that the primary data source is switched when its accuracy falls below an acceptable level, regardless of the range or classification of the monitoring parameter values generated by the secondary data source.

If the accuracy of source A is not below the threshold, source A remains the primary data source, and the process continues. This allows the method to maintain the current primary data source as long as its accuracy remains above the threshold, thereby minimizing unnecessary switches between data sources.

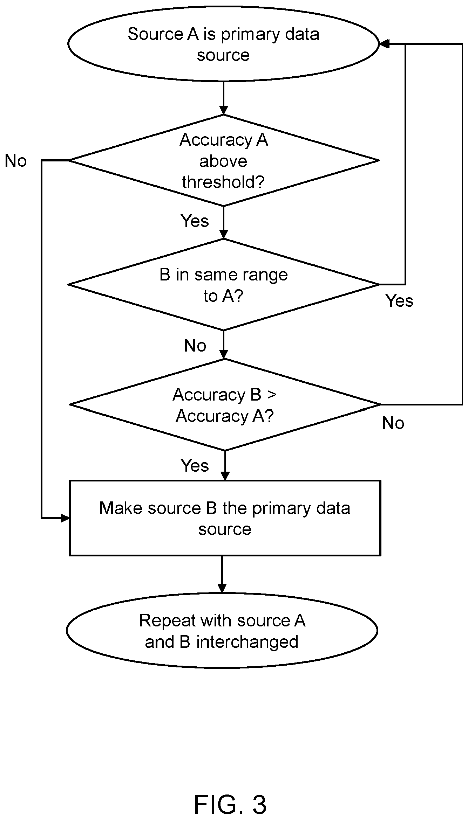

Referring to FIG. 3, another specific method for selecting a primary data source among multiple data sources is depicted. In some aspects, the method begins with source A designated as the primary data source. The process involves comparing the accuracy of source A to source B. The accuracy of each data source may be determined based on various factors such as the quality of the data generated by the respective data source, the reliability of the data source, and other factors that may affect the accuracy of the data.

In some cases, the method checks if the accuracy of source A is above a threshold. The threshold may be a predetermined value or range of values that represents an acceptable level of accuracy for the data source. If the accuracy of source A is above the threshold, the method checks if source B is in the same range as source A. The range may refer to a specific range of values for the monitoring parameter, a classification of the monitoring parameter, or any other categorization of the monitoring parameter values. The determination of whether source B is in the same range as source A may be based on various factors such as the values of the monitoring parameter generated by the respective data sources, the accuracy of these values, and other factors that may affect the categorization of the monitoring parameter values.

If source B is in the same range as source A, in some aspects, the process loops back to the beginning. This allows for a smooth transition between data sources, minimizing potential confusion or misinterpretation of data by healthcare professionals.

If source B is not in the same range as source A, the method checks if the accuracy of source B is greater than that of source A. If the accuracy of source B is greater than that of source A, source B is made the primary data source, and the process repeats with source A and source B interchanged. This ensures that the primary data source is switched when a secondary data source provides more accurate data, so that the range or classification of the monitoring parameter values that is used is as accurate as possible.

If the accuracy of source A is not above the threshold or if the accuracy of source B is not greater than that of source A, the process loops back to the beginning. This allows the method to maintain the current primary data source as long as its accuracy remains above the threshold and is not surpassed by a secondary data source, thereby minimizing unnecessary switches between data sources.

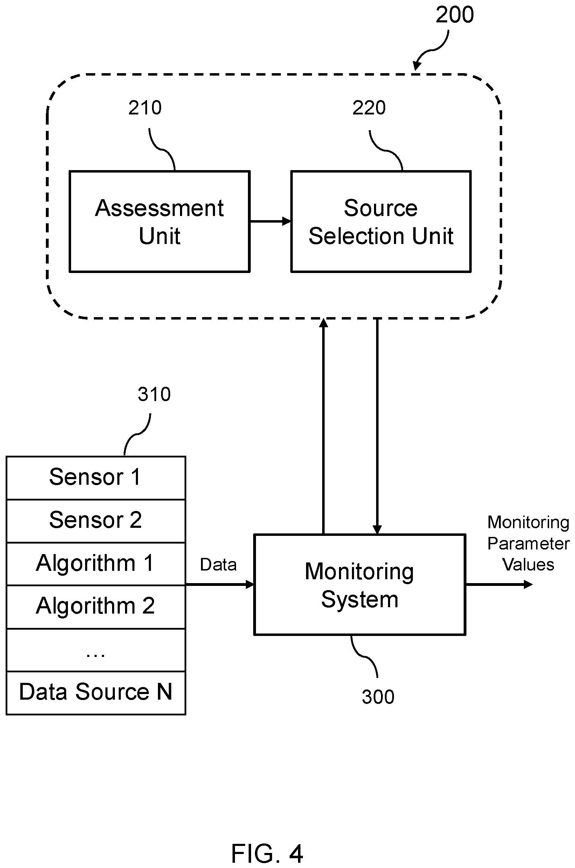

Referring to FIG. 4, a system 200 for selecting a data source amongst a plurality of data sources 310 is depicted. Each data source is configured to generate data suitable for determining a value of the same monitoring parameter of a subject. The plurality of data sources 310 may include a primary data source that is a currently selected source of the monitoring parameter value and at least one secondary data source that is not a currently selected source of the monitoring parameter value.

The system 200 includes an assessment unit 210 and a source selection unit 220. The assessment unit 210 is configured to determine a fitness score of each of the plurality of data sources 310. The fitness score may be based on various factors such as the accuracy of data generated by the respective data source, patient comfort information, system footprint information, data delay information, and future clinical suitability information associated with the respective data source.

The source selection unit 220 is configured to select an updated primary data source amongst the plurality of data sources 310 based on the fitness scores. The selection of the updated primary data source is biased towards maintaining the primary data source as the current source of the monitoring parameter. This biasing towards maintaining the primary data source can help minimize potential confusion or misinterpretation of data by healthcare professionals.

Of course, any of the methods described above for determination of the fitness scores and/or selection of the updated primary data source may be implemented by the system 200.

In some aspects, the system 200 may use a virtual sensor defined as a combination of two different sensors. For instance, a third virtual sensor (sensor 3) can be defined as a combination of sensor 1 and sensor 2. Due to the nature and origin of the artifacts in the ECG signal (example artifacts due to muscle electrical activity) sensor 1 and sensor 2 signals can be noisy, but sensor 3 can be clean. Following the logic of the system 200, the bipolar sensor 3 can be selected as a primary sensor, and can be used until computational resources are saved and usage of unipolar sensors becomes feasible. This allows the system 200 to leverage the strengths of multiple sensors to provide more accurate and reliable monitoring parameter values.

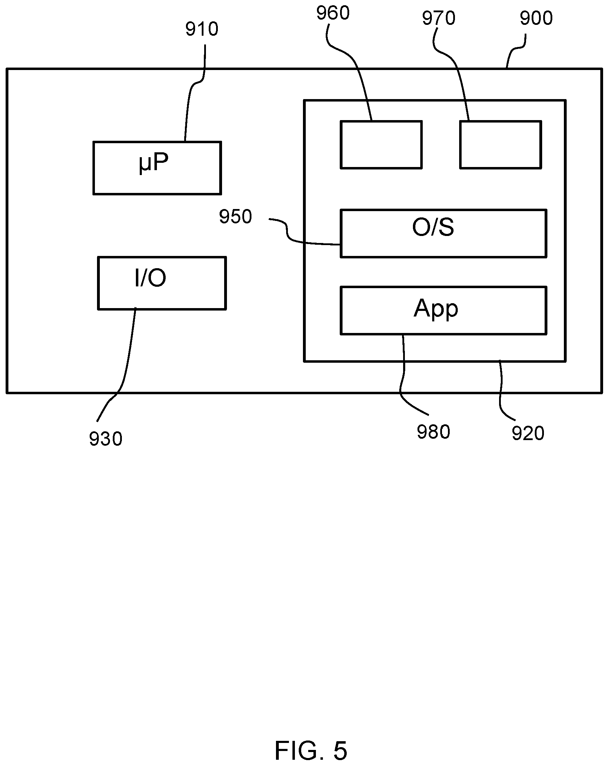

FIG. 5 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for selecting a data source amongst a plurality of data sources, wherein each data source is configured to generate data suitable for determining a value of a same monitoring parameter of a subject, and the plurality of data sources comprises a primary data source that is a currently selected source of the monitoring parameter value and at least one secondary data source that is not a currently selected source of the monitoring parameter value, the method comprising:
determining (110) a fitness score of each of the plurality of data sources;
selecting (120) an updated primary data source amongst the plurality of data sources based on the fitness scores, wherein selecting the updated primary data source is biased toward maintaining the primary data source as the current source of the monitoring parameter.

2. The method of claim 1, wherein selecting (120) the updated primary data source comprises:
responsive to the fitness score of one of the secondary data sources exceeding the fitness score of the primary data source, assessing (122) a switching criterion describing a condition during which switching of the currently selected source of the monitoring parameter value is facilitated; and
responsive to the switching criterion being met, selecting (130) the secondary data source as the updated primary data source.

3. The method of claim 2, wherein the switching criterion comprises a fitness variance threshold, the fitness variance threshold imposing that the score of the secondary data source exceeds the fitness score of the primary data source by a predetermined difference.

4. The method of claim 2 or 3, wherein the switching criterion comprises a value variance threshold, the value variance threshold imposing that a difference between the value of the monitoring parameter determined based on data from the secondary source and the value of the monitoring parameter determined based on data from the primary source is less than a predetermined difference.

5. The method of any of claims 2-4, wherein the switching criterion comprises a fitness failsafe threshold, the fitness failsafe threshold imposing that the fitness score of the primary data source fails to meet a predetermined condition.

6. The method of any of claims 2-5, wherein the switching criterion is based on a medical context of the subject, a status and/or condition of the subject, an alarm context, and/or a workflow context.

7. The method of any of claims 1-6, wherein the fitness score of each data source is based on a confidence score indicating an accuracy measure of data generated by the respective data source and/or an accuracy measure of the value of the monitoring parameter determined based on data from the respective data source.

8. The method of claim 7, further comprising determining the confidence score based on analyzing data from the data source, and/or based on analyzing data source context information.

9. The method of claim 8, wherein the data source context information comprises expected source quality information, historical source quality information, sensor source placement information, sensor source use information, source interference information, and/or perceived source quality information.

10. The method of any of claims 1-9, wherein the fitness score of each data source is based on at least one of patient comfort information, system footprint information, data delay information, and future clinical suitability information associated with the respective data source.

11. The method of any of claims 1-10, wherein selecting (120) the updated primary data source is further based on primary data source historical use information.

12. The method of any of claims 1-11, further comprising:
responsive to selecting the updated primary data source, delaying switching (130) the currently selected source of the monitoring parameter values to the updated primary data source based on contextual use information associated with the monitoring parameter values.

13. The method of any of claims 1-12, further comprising at least one of displaying the values from the primary data source; generating an interface output based on the values from the primary data source; generating an alarm based on values from the primary data source; providing the values from the primary data source of the monitoring value to a clinical algorithm; and/or modifying an operating state of secondary data sources.

14. A computer program comprising computer program code means adapted, when said computer program is run on a computer, to implement the method of any of claims 1-13.

15. A system (200) for selecting a data source amongst a plurality of data sources (310), wherein each data source is configured to generate data suitable for determining a value of a same monitoring parameter of a subject, and the plurality of data sources comprises a primary data source that is a currently selected source of the monitoring parameter value and at least one secondary data source that is not a currently selected source of the monitoring parameter value, the system comprising:
an assessment unit (210) configured to determine a fitness score of each of the plurality of data sources; and
a source selection unit (220) configured to select an updated primary data source amongst the plurality of data sources based on the fitness scores, wherein selecting the updated primary data source is biased toward maintaining the primary data source as the current source of the monitoring parameter.
